# EUROPEAN PATENT SPECIFICATION

(11) **EP 0 539 383 B1**
(45) Date of publication and mention of the grant of the patent: **18.09.1996**
(21) Application number: 91910056.0
(22) Date of filing: 20.03.1991
(51) Int. Cl.: G01N 21/00, G01N 21/75, G01N 33/552

(54) **APPARATUS FOR DETECTION OF AN IMMOBILIZED ANALYTE**
APPARAT ZUM NACHWEIS EINES IMMOBILISIERTEN ANALYTEN
APPAREIL DE DETECTION D'UN ANALYTE IMMOBILISE

(43) Date of publication of application: 05.05.1993
(73) Proprietor: BIOSTAR, INC., Boulder, Colorado 80301-3325 (US)
(72) Inventor: ETTER, Jeffrey, B., Boulder, CO 80302 (US); MAUL, Diana, M., Denver, CO 80221 (US); MODDEL, Garret, R., Boulder, CO 80304 (US); STARZL, Timothy, Boulder, CO 80304 (US); HANLIN, H., John, Louisville, CO 80027 (US)
(74) Representative: Senior, Alan Murray
(86) International application number: US9101781
(87) International publication number: WO9216826

(56) References cited:
- EP-A- 0 276 968
- US-A- 4 054 646
- US-A- 4 090 849
- US-A- 4 558 012
- US-A- 4 647 544
- US-A- 4 654 300
- US-A- 4 737 464
- US-A- 4 921 878
- US-A- 4 931 384
- DATABASE WPI Week 8907, Derwent Publications Ltd., London, GB; AN 89-048044 & EP-A-0 303 229 (E. ISHIKAWA)
- PATENT ABSTRACTS OF JAPAN vol. 14, no. 349 (P-1048)27 July 1990 & JP-A-02 124 462 (MITSUI TOATSU CHEM.) 11 May 1990
- CHEMICAL ABSTRACTS, Vol. 96, issued 1982, Fugitsu Ltd, "ETCHING" see Abstract No. 96: 96322 N, page 742 JP 81,144,541.

## Description

### FIELD OF THE INVENTION

This invention relates to the field of biochemistry and chemistry, with applications in such areas as immunoassay, nucleic acid sequence analysis, clinical chemistry, enzyme assay, and the study of ligand/anti-ligand binding pairs such as toxin-receptor or hormone-receptor combinations, etc. This invention utilizes a novel solid phase assay based on optical interference, a direct physical detection method. In this solid phase assay, the receptive material is immobilized on a coated support surface. The immobilized receptive material is exposed to a fluid that may contain the analyte, which is a molecule of interest capable of binding to or reacting with the receptive material. The analyte can be any material for which a specific reactive partner is available, either organic or inorganic. In general, the roles of the receptive material and the analyte in the assay can be reversed.

### PRIOR ART

Assays exist in the art for monitoring reactions between homologous couples such as antigen-antibody binding, hormone-receptor binding, enzyme assays, and nucleic acid hybridization. The critical part of assay design in each case is deciding how the signal will be generated. Colored reaction products, biological activity at the cellular level, and radioactive, fluorescent, or luminescent tags covalently bound to one of the reacting species have all been used. Two familiar examples in the field of immunoassay are enzyme-linked immunosorbent assay (ELISA) and radioimmunoassay (RIA). In these methods, the experimenter does not actually detect the binding of the two molecules, but some secondary activity related to the binding reaction. Many of these techniques require special steps for signal generation, resulting in added labor costs, and often requiring use of hazardous materials and expensive instrumentation.

A more advantageous means of detecting binding or reaction between two species of biological molecules is to detect a change in some physical property that is a direct result of the binding or reaction. For example, if the ligand is present as a thin film immobilized on a substrate, then any method that can measure a change in thickness or optical mass of the biological thin film after binding or reaction of the analyte with the ligand would be a means for direct physical detection. This invention provides such a method, based on the principles of optical interference in thin films.

Optical detection techniques have existed in biochemistry since the middle part of this century, especially in the area of immunoassay. All of these techniques utilize: a solid support or substrate; intermediate layers that may be necessary for generating the signal; a thin film (usually monomolecular) of a biologically active molecule, the ligand; and an optical component comprising of a source of radiation, a means for setting the angle of incidence, if necessary, and a means of detection. The various optical assays measure different properties of light, use different substrates, and are of varying degrees of utility in different environments (research laboratory vs physician office laboratory vs home or field use).

The first solid-phase immunoassay using direct optical detection was based on the Langmuir-Blodgett technique. This technique provides a means for producing monolayers of amphipathic molecules on the surface of water, then transferring these films to a glass or metal substrate. Multilayer films of fatty acids can be built up on the substrate by multiple passages of the substrate through the monolayer. These monolayers could then be used as supports for adhesion of monolayers of proteins (K.B. Blodgett, JACS, 56, 495, 1934). In the published immunoassay (I. Langmuir and J.J. Schaefer, JACS, 59, 2400, 1937), a barium stearate film of approximately fifty monolayers was produced on a chromium plated glass slide. The barium stearate film was then coated with a layer of diphtheria toxin, which added 3.6 nm to the thickness of the film. The ligand-coated slide was then dipped into a solution containing anti-toxin antibodies. After incubation with the antibodies, the thickness of the film increased an additional 7.5 nm. In this experiment, the thickness changes were measured by illuminating the slide with s-polarized light at large angles of incidence and determining the reflectance minimum. This detection system is sensitive enough to measure thickness changes as small as 1 Angstrom. Modification of this technique have been discussed by Kawaguchi, U.S. Patent 4,820,649 wherein a metal substrate was coated with lipids to produce a specular interference effect. The resultant product, however, is very difficult to evaluate as the viewing is highly angle dependent. The specular interference color generation is weak.

Although very sensitive tests are possible, assays based on the Langmuir-Blodgett technique are no longer of practical importance in biochemistry because of several serious limitations. The technique is very difficult and time-consuming, and requires reagents, especially water, of very high purity. It is also very difficult to produce lipid coated substrates. Any perturbation of the water results in flaws in produced lipid monolayers.

The principle of optical interference through the superposition of light waves with varying phase relationships and amplitudes has been used to monitor biological reactions. The first optical assays using interference colors as a means of detection used metal or metallized substrates, with the ligand molecule simply absorbed to the surface. For example, A.L. Adams, et al. J. Immunol. Methods, 3, 227, 1973, used anodized tantalum with a tantalum oxide layer. Giaever (1974) U.S. Pat. 4,054,646, used indium coated onto glass slides. These substrates have numerous disadvantages. In particular, Giaver's surfaces are difficult to reproducibly manufacture. The metal surfaces are also disadvantageous from the chemistry point of view. Metal ions can leach from the surface and interfere with the chemistry of binding between the ligand and the analyte. Furthermore, the surfaces of such substrates are not as versatile for organic, covalent linkages as a silica or glass surface. Giaver's technique relies, in part, on the generating of an interference effect through a diffraction-scattering effect and in part on the metal's absorptive properties.

Two more recent inventions use reflectance or optical interference effects to detect changes in thin films caused by analyte binding. One method monitors a change in thickness and the other measures at the changes in polarizability of immobilized thin films. H. Arwin and I. Lundstrom, Anal. Biochem., 145, 106, 1985 use a method that is reminiscent of the original work by Langmuir, Blodgett, and Schaefer. They used a silicon substrate, with a thermally grown 10 nm thick silicon dioxide layer. The surface was made hydrophobic with the use of dichlorodimethylsilane, and in the published example, human serum albumin was adsorbed onto the hydrophobic surface, presumably as a monolayer. The invention exploits the reflectance properties of p-polarized and s-polarized light. At the Brewster angle, which is determined by the refractive indices at the boundary between two media, no p-polarized light is reflected. When the substrate is an absorbing material and the medium is transparent as in this case, the reflectance minimum is shifted to the so-called pseudo-Brewster angle. At this angle of incidence, p-polarized light has measurable, but minimal reflectance. When the thickness of the dielectric layer increases, for instance due to antibody binding to immunological reaction, there is an increase in reflectance at the pseudo-Brewster angle. The method is simple in concept, but requires extremely precise instrumentation in practice. First, a polarizer must be used to obtain and isolate the polarization component of light. For use with a silicon substrate, the incident light must be further processed with a filter to eliminate red wavelengths. Other substrates may require monochromatic or near-monochromatic light. Thus, the instrument is not as flexible or adaptable as other optical detection instruments. Two photodiodes are required: one to measure reflected intensity and one to measure the intensity of the incident light. An electronic processing unit containing an amplifier and a display are also needed. Most importantly, the angle of incidence must be measured very precisely, so all components and the sample must be mounted in a precision machined housing. This published account focused specifically on an immunoassay format.

Another related method is disclosed by Nicoli, et al. in U.S. Patent 4,647,544. This patent discloses a method for immunoassay using the principle of optical interference. The interference effect used in this invention is a change in light intensity at various point in space rather than a change in interference color and which is not capable of generating a visually interpretable signal. In particular, this method detects constructive interference of light at Bragg scattering angles using a diffraction grating to produce scattering. A crucial part of this invention is the preparation of the surface. The ligand (e.g. antibody) is applied as a regular array on the substrate. In the alternative, the ligand is applied in a uniform fashion and then inactivated in the precise pattern required to create a diffraction grating. The preferred embodiment is to apply the ligand in strips of width w and center-to-center spacing d, where d is greater than w. Both d and w are of microscopic dimensions. The regular array acts as a diffraction grating, and maximal scattering occurs at angles Θₛ, where: d sin Θₛ = m λₛ (m = 1,2,3,...). The important physical property measured in this scheme is a change in polarizability relative to the medium. When an antigen binds to the immobilized antibody, there is an increase in polarizable mass at regular intervals in the array, and the intensity of light at angles Θₛ increases as well. As this method of detection observes second order effect, there is a reduction in assay sensitivity relative to other interference phenomena. Production of the arrays of ligand to the tolerances required for this technique greatly increases manufacturing costs and the rejection rate of initial test surfaces.

In another embodiment, Nicoli et al coat an actual diffraction grating with the ligand and detect Bragg scattering. In both embodiments, the peaks of scattered light can be detected in either the reflected or transmitted light depending on the nature of the substrate.

As in the method of Arwin and Lundstrom, special light sources and optical equipment are necessary to detect the interference effects. Since only intensity changes due to optical interference are being detected, a monochromatic light source must be used. In the disclosed example, a He/Ne laser served as the light source. Detection at the scattering angle is achieved with a device such as a photomultiplier tube or solid state photodiode. As signal is scattered, a large number of detection angles are possible but sensitivity suffers, as no single measurement is adequate.

The technology most closely related to the present invention was first disclosed by Nygren, et al. (1985) in U.S. Patent 4,558,012. This patent covers a method and a device for optical interference assays that allow for direct detection of results using a non-metal substrate and a dielectric film. The dielectric film is made up of a layer of silicon nitride or silicon oxide with a top layer of SiO₂ (1.46) is very close to that for most organic molecules (n = 1.5), so that the two substances can function as a single layer for optical purposes. The thicknesses are chosen such that the entire dielectric film can act as a single layer anti-reflection coating, optimized for a wavelength present in the polychromatic incident light, giving the unreacted slide a characteristic interference color. When the analyte binds with the ligand, the optical pathway changes, giving a new wavelength minimum in the reflected light and a new visible interference color. Use of a polychromatic light source means that an interference color is seen in the reflected light rather than interference fringes or an intensity change. This technique depends strongly on the specular reflective properties of the substrate.

A similar technology is described in these Teijin patent applications: Japan Patent Application SHO 61-222057, and SHO 61-222058; and the Teijin European patent application 87113842.6 shows a method to enhance the visible interference signal.

Most solid phase systems use support surfaces that appear macroscopically planar and uniformly. However, on microscopic examination, these surfaces are highly convulated and irregular. Generally, the characteristics of the surfaces are not critical to the production of the assay signal. The surface characteristics may impact the density of ligand in the assay. The optical detection systems described above rely on patterned, arrayed, or grated substrates for generation of scattering or signal. These optical systems also use patterned or arrayed ligand coatings to affect the signal.

Methods for physically roughening or etching the surface of a silicon substrate are known in the electronics and semiconductor industries. Glass and silica surfaces may be etched with modifications of these techniques. Anisotropic etching of silicon, gallium arsenide, and germanium provides means for introducing structure or texture onto the substrate surface. Potassium hydroxide or sodium hydroxide are commonly used in etching protocols. The etch rate and depth can be controlled by varying the concentration of the alkaline solution and using selected additives.

For example, I. Stoev and L. Petkanov Bulg, J. Phys., 9(3), 277, 1982 describe the use of n-propanol and hydrazine in KOH solutions to decrease the etch rate and reduce defects in the manufacture of VMOS structures. Erdman, et al. (Ger Offen 2,245,809) describe the use of H₂O₂, K₂CrO₄, K₂CrO₄ and tetrahydrofurfuryl alcohol as additives in alkaline etching solutions. Another aqueous etching system described by Petkanov for production of VMOS structures is a combination of ethylenediamine-pyrocatechol-water. Dry etching procedures are also available. Japanese patent JP 81,144,541 teaches the use of an etchant gas containing C₂F₅C1 and CF₂ generated in a plasma environment. J.A. Barkanic, et al., EP 246514A2, 1987, use mixtures of NF₃ in CF₃C1, CF₂C1, and CF₃Br in a plasma generator. Patterns can be created in this plasma environment by masking discrete sections of the surface with SiO₂ which is not susceptible to the etchant gases.

It is an object of this invention to provide an assay using an optical detection method, and more specifically the generation of interference colors, to monitor binding and/or reaction between two molecular species--the ligand and the analyte.

It is still another object of this invention to provide a simple and inexpensive assay used at home and in the laboratory capable of selectively attaching an analyte of interest and generating a corresponding binding signal which is based on instrumented detection making optional, visible signal generation.

A still further object of this invention includes an improvement in optical interference assays employing an assay device having an irregular surface which produces a diffuse reflection. As will be seen hereafter, the surface imperfections or irregularities are not regularly spaced as in a diffraction grating, and the random variations in height can be in the range from hundreds of nm to about 100 microns.

The present discovery makes use of the fact that the irregular surface serves three functions. The first of these is to increase the surface area available for coating with receptive material, and resulting in a concomitant increase in reaction speed and/or sensitivity of the assay. The second function is that the interference colors are seen in diffuse rather than specular reflection. This means that, in contrast to a diffuse reflection when interference colors are seen against a specular reflecting surface, images of distant objects are also visible in the reflected light. These images can confuse the viewer or even cover up color spots, especially if the color is faint and the assay is near the limit of detection. The third functional advantage is that a diffuse reflection allows visible color change over a broad range of angles of incident light. Specular reflections, in contrast, are dependent on the angle of incident light. If the article is incorrectly positioned with reference to the light source, the color change becomes indistinct.

The device which embodies the present invention also provides a versatile assay technology. This invention can be made from a wide variety of substrates, anti-reflective (AR) materials, and receptive materials. The proper selection of material components allows the test to be formatted to generate a signal appropriate for a wide range of testing needs. While most home use assays simply require qualitative (positive vs. negative results), use in the practitioner's office often demands a more quantitative result to diagnose or monitor therapeutic efficacy; the various embodiments of this invention provide for this type of format versatility beyond immunoassay which includes DNA·DNA, DNA-mRNA, DNA·RNA, RNA·RNA, enzyme substrate, enzyme inhibitors and the like.

Healy *et al*, US Patent No. 4090849 describes an assay system where a metal substrate is surface roughened, chemically etched and the etched surface is anodized to produce a dull, oxide surface. Metal substrates have the disadvantage of impeding reflection of light by absorbing it. The present invention relies on anti-reflective properties involving changes of dielectric film.

The present invention provides a device for detecting the presence of an analyte of interest comprising:
a substrate supporting or having attached thereto, a layer of an analyte-receptive material, constituting in combination with an anti-reflective coating an optically active surface exhibiting a first interference color in response to light impinging thereon, and exhibiting a second interference color comprising a combination of wavelengths of light different from said first interference color or comprising an intensity of at least one said wavelength of light different from said first interference color, in response to said light when said analyte is present on said optically active surface;
wherein said substrate has a non-specular surface or wherein a transparent layer having a non-specular surface is provided for viewing of said optically active surface.

The present invention includes novel compositions and novel articles for the direct selective absorption, adsorption, covalent binding or attachment by whatever mechanism, of an analyte, specific to the receptive material bound to the surface of the apparatus for the purpose of identification or quantitation of the analyte. The present invention in its broadest sense provides a method of detection of an analyte without the use of labels such as radioactive enzyme, fluorescent, or luminescent conjugates and the like, i.e. using unlabelled detection materials. This method of constructing the detection device comprises the steps of depositing one or more anti-reflective (AR) coatings onto a substrate material, covalently binding, affixing or adsorbing, receptive material specific to the analyte of interest to the top layer of anti-reflective material. The optical substrate coated with receptive material is then contacted with a fluid containing the corresponding analyte of interest and examined for a diffuse reflection, produced by the coated article, to determine whether a colored spot or other suitable optical signal appeared. In one embodiment of this invention, the substrate has an irregular surface, therefore the reflection is diffuse. In another embodiment, however, the reflection is initially specular, but the reflection is then made non-specular by means of light reducing, light scattering, light diffusing, light attenuating, or light modifying, material such as smoked glass or textured plastic which is placed over the article to produce a non-specular reflection.

The disadvantages of many specular assays intended for use as non-instrumented visual tests include that the viewing must be done through polarizers or filters, so that the assay's sensitivity is limited by the dependence on the angle of incident light. For example, a substrate with a refractive index of 2.25 coated with an anti-reflective layer with a real refractive index of 1.50 has a lack of sensitivity when the incident light is 30 degrees or more from perpendicular incidence (T. Sandstroem, M. Stenberg, and H. Nygren, Applied Optics, Vol. 24, pg. 472-479 [2/15/85]). It is unexpected and surprising to find that an irregular substrate, or light diffusing material, placed over the article, would create a diffuse reflection that would reduce or eliminate the incident light angle dependence of the article and still reveal a clear signal, i.e. a change in interference color. The present invention employs a method and article that produce a diffuse or non-specular reflection which is a substantial improvement over the assay detection methods previously known.

According to the preferred practice of this invention, a substrate is selected to have some of the various attributes described herein. The substrate can be formed of a reflective material such as polished silicon, polished metals, etc., or with little difficulty the assay can be performed on a transmissive substrate such as certain plastics, glass, quartz, etc. The substrate may be a solid support, a flexible support, a pellicle or a gel. Examples of some suitable materials for making a substrate are metal, quartz, plastic, silicon, non-metals or functionally equivalent materials capable of having anti-reflective material coated onto the surface.

A flexible support is especially useful for articles that are marketed as home test kits. When shipped, these articles do not require special handling or expensive protective packaging. Examples of some of the materials which are suitable for forming flexible supports are various forms of plastics, or malleable metals, etc. The substrate can also be a pellicle which is both lightweight and flexible, or the substrate can be formed of a gel-like substance. Furthermore, the substrate can be any substance onto which is coated a layer of material capable of receiving an anti-reflective coating and meeting the criteria hereinafter described. The critical criterion for the choice of substrate material is its coatability with an anti-reflective material, and its refractive index is approximately equivalent to the square of the refractive index of the material directly above it. A wide variety of ratios between the refractive indices of the substrate and the material directly above it can be utilized within the scope of this invention. It is also important for signal generation that the interference color produced by the pre-formed article and the interference color produced by the reacted article should be visually contrasting to yield a highly visible signal.

According to a preferred practice of this invention, the substrate of the inventive device has an irregular surface. The surface imperfections are not regularly spaced and the variations in height determined by profilometry are on the order of 200-300 nanometers to about 100 micrometers. Methods for generating an irregular surface include anisotropic and isotropic etching of the surface. In some cases, the nature of the intrinsic substrate material renders the surface irregular. Suitable surfaces are also generated using aluminum oxide or other lapping particles of the appropriate diameter to abrasively create the diffuse surface. However, it should be understood that some of the embodiments of this invention utilize a smooth substrate surface as the preferred substrate.

After selection of the appropriate substrate, an anti-reflective coating is affixed to the substrate. The anti-reflective coatings are deposited onto the surface of the substrate by known coating techniques, for example, sputtering, vapor phase deposition in a vacuum chamber and the like. Material useful as anti-reflective coatings have four attributes, clarity, essentially colorless at the thickness utilized, room temperature stability, sufficient stability to withstand the deposition techniques, and capable of suppressing reflective light at selected wavelengths when coated. The function of anti-reflective material includes an interference color, and second to providing a layer onto which receptive material can be affixed. The receptive material's capacity to selectively adsorb or bind any analyte of interest present in the fluid to be contacted with the apparatus must not be adversely affected by the immobilization process.

The anti-reflective material in this invention produces an interference color by destructive interference of certain wavelengths of light; for example, an anti-reflective layer of silicon monoxide coated at a thickness of 600 Angstroms on a silicon substrate produces a bluish interference color. Silicon dioxide on a similar substrate and at a similar thickness produces a gold color.

The thickness of the coated anti-reflective material is dependent on whether monochromatic or polychromatic light will be utilized to radiate the surface of the assay apparatus, and the degree of sensitivity desired in the assay. The greatest reduction in reflection occurs for an anti-reflective coating having a thickness of approximately one-fourth of the wavelength of light in the medium, and a refractive index that is the square root of the product of the indices of the media directly above and directly below it.

An article optimized to be illuminated with polychromatic light should have an AR coating of a thickness of one quarter of the wavelength selected for suppression. If the article is to be illuminated with monochromatic light and a high degree of sensitivity is desired, then the device should be coated with one or more anti-reflective layers with an optical thickness on the order of several wavelengths. This serves to further optimize wavelength suppression for a selected wavelength. For example, if the device of the present invention was coated with a thick anti-reflective layer (e.g. 1 µm) and illuminated with a fixed wavelength (e.g. 6328 Angstroms from a HeNe laser) an extremely small change in the thickness of layers on the test device will produce a significant change in the wavelength of the reflected or transmitted light.

A reflective substrate coated with an optical thickness of one to several wavelengths of anti-reflective material may utilize the same optical principles as a Fabry Perot interferometer. The anti-reflective materials form a well with a reflecting top and bottom surface that allows only certain optical wavelengths to exist therein. The reflected wavelengths from this article vary strongly as a function of wavelength of incident light. The thicker the anti-reflective layer or layers, the higher the Q of the cavity, and the narrower the band of reflected wavelengths will be, thus increasing the sensitivity of the device. This article when illuminated with polychromatic light shows substantial changes in reflected wavelength with thickness changes of only a few Angstroms.

After affixing the anti-reflective layer or layers to the substrate, receptive material is attached to the uppermost anti-reflective layer by various means. The uppermost anti-reflective layer can be chemically activated to allow covalent binding of the receptive material, or to provide adsorption of the receptive material. The receptive material can be coated onto the anti-reflective material by the Langmuir-Blodgett method. The function of the receptive material is to selectively adsorb, bind or affix, by various means, a specific analyte from any fluid containing the analyte.

The precoated device or article may then be utilized to assay any fluid suspected or believed to contain the analyte of interest. The suspect fluid is exposed to the precoated device, and is allowed to incubate for a predetermined period of time. Next, the device is rinsed to remove unbound material and dried. A change in the visible interference color is evidence of a positive result while negative results exhibit no change in the interference color.

In the following examples, the uppermost layer of anti-reflective material onto which the receptive material is coated is a silicon oxide; however, any anti-reflective material capable of adhering receptive material is a suitable substitute. Examples of top anti-reflective layers include, but are not limited to, elemental carbon (in graphite-like or diamond-like lattice structures), boron compounds, organic polymers TiO₂, alumina, silicide compounds, or any other compounds that exhibit the ability to be activated to attach, adhere, affix or otherwise secure receptive material which is capable of selectively binding or interacting with the analyte in any sample fluid.

Subsequent qualitative or quantitative detection of the analyte is made easily since the reflection is diffuse and less angle dependent. Qualitative detection of the analyte is by the unaided eye, quantitative detection is performed with such instruments as the Sagax® (registered trademark of BioStar Medical Products, Inc., Boulder, Colorado) comparison ellipsometer or a reflectometer, or any other instrument capable of measuring such physical parameters as wavelength, polarization, intensity, small optical density changes, etc.

### BRIEF DESCRIPTION OF THE DRAWINGS

Figure 1 represents a prepared slide with an irregular surface, anti-reflective layers, receptive material;
Figure 2 represents a prepared slide reacted with analyte;
Figure 3 represents the diffuse or non-specular reflection of light from an uneven substrate;
Figure 4 represents the specular reflection of light from a planar substrate and the non-specular reflection of light from a planar substrate covered with plastic;
Figure 5 represents a prepared slide, coated with receptive material and partially covered with a non-specular material; and
Figure 6 represents a prepared slide with an irregular surface and receptive material.

### DETAILED DESCRIPTION OF THE INVENTION

The following definitions of terms used herein are supplied for the purpose of clarifying aspects of the invention and are not intended to limit the invention in any way and are believed to be art-accepted:

Anti-Reflective Layers: A thin layer of material coated on a substrate to suppress unwanted reflections from the surface. The layer is most effective when the reflections from the upper surface of the layer and the upper surface of the substrate are approximately 180 degrees out of phase with each other.

Antibody: A class of serum proteins which specifically bind and whose production was induced by an antigen.

Antigen: Molecules which induce an immune reaction when recognized by the host's immune system.

Interference Phenomena: A destructive and/or constructive interaction of two or more lightwaves resulting in an intensity that has a different resulting sum than the sum of the original component waves.

Diffuse Reflection: Incident light reflected from a surface that has surface structures which are large in comparison to the wavelengths of irradiated light.

Specular Reflection: Incident light reflected from a surface that has surface structures which are small in comparison to the wavelengths of irradiated light.

Index of Refraction: Ratio of speed of visible light in a material to the speed of light in a vacuum.

Optical Thickness: The measurable physical thickness of a material multiplied by the index of refraction of the material.

Lapping: The process in which abrasive particles are used to mechanically produce surface structures. The abrasive particle size will determine the character, size and distribution of surface features.

Etching: A chemically controlled process for the production of surface features on a substrate.

Interference Color: The phenomena of one or more wavelengths of light constructively or destructively interfering, and thus suppressing certain wavelengths in a region of the spectrum and reflecting a color by an interference phenomenon.

What follows is a description of the invention including the steps of coating the substrate with anti-reflective material and receptive material to form the precoated device, the method of assaying for the analyte in the fluid sample, and the method for detecting and determining the amount of analyte bound to the substrate. The use of markers or labels such as radioactive, enzyme, fluorescent, luminescent conjugates, etc. are obviated by this unlabeled device. To elaborate the description of the invention, a specific example was selected. A silicon wafer served as the substrate. Silicon monoxide and silicon dioxide were the coated anti-reflective layers and silane chemistry was utilized to bind bovine gamma globulin, the receptive material, to the device. The method of preparing the device is not limited to the specific materials selected in this example. Any compounds which fit the specific criteria listed for each of the elements of the apparatus or device can be utilized by art-accepted and known means of slight changes to the hereinafter described process.

### STEP 1: Substrate Selection

This invention allows diverse substrate formats; the substrate can be a support which is solid, flexible, semi-flexible, a pellicle or a gel depending on the type of analyte chosen and the assay characteristics desired by the end user.

Preferred embodiments of the present invention include use of a substrate with an irregular surface to produce diffuse light reflection; and a smooth substrate surface with a light diffusing or light modifying material such as smoked glass or textured plastic. This is applied over the slide after the apparatus has reacted with the analyte. The first embodiment is formed with a substrate of suitable material with at least one irregular surface (hereinafter referred to as the irregular substrate). A simple method of generating an irregular surface is to use material with a lattice structure that forms an irregular pattern when cleaved. However, material with a lattice structure that forms a smooth surface when cleaved can also be utilized in the first embodiment (and obviously without further change in the second embodiment) of this invention. A smooth substrate surface can be treated by a variety of processes in order to form an irregular substrate. These processes include, but are not limited to: anisotropic etching, isotropic etching, abrasive lapping, HF acid treatment, and chemical/plasma etching to create the surface features which provide a diffuse substrate.

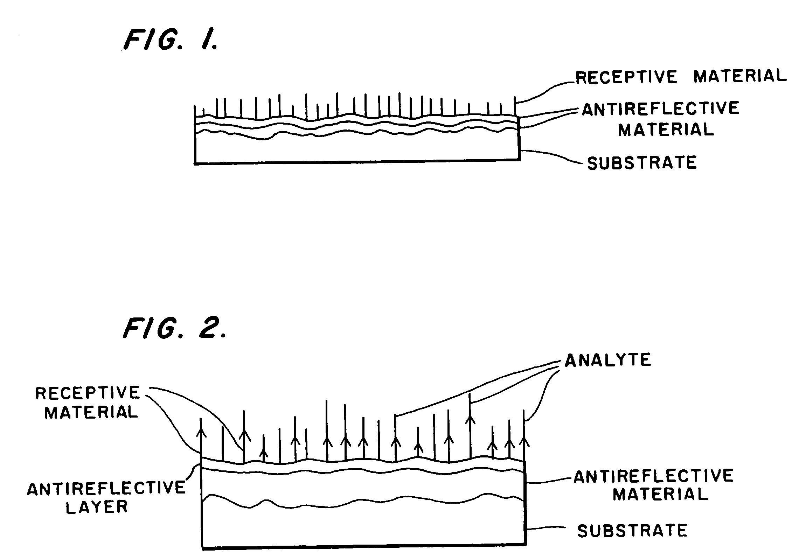

Referring to Fig. 1, there is shown a substrate having a substantially irregular surface fabricated of any suitable material; in this figure the selected substrate is a silicon wafer. The wafer is approximately 4 inches in diameter and .02 inches thick; it should be noted that these dimensions of the substrate are not critical to the present invention.

The substrate is formed from a silicon crystal which is grown and extruded to 4 inches in diameter and then diamond sawed to form a wafer. The wafers are treated with chemical etchants to smooth the surface and reduce flaws. The wafers are lapped or ground with aluminum oxide or titanium oxide particles in a talc slurry. The initial grain size is large and successively smaller particle sizes are used to produce an increasingly smoother surface. Both sides of the wafer are subjected to this process. The final lapping process leaves a very diffusely reflective surface. The wafers may then be used in this application after the anti-reflective coatings are applied. Wafers may be further processed with chemical or plasma etching to modify the diffuse reflecting characteristic of the substrate. Wafers in the semi-conductor industry undergo additional processing to be polished on one or two sides and are of use in the second embodiment of this invention.

Once the wafers are lapped, they are cleaned using the following process or a known modification thereof: the wafers are sonically cleaned with a cationic detergent, followed by a rinse in 18 mega ohm water. Then they are cleaned with anionic detergent, followed by a rinse in 18 mega ohm water. Then they are ultrasonically cleaned with an aqueous ammonia solution made of 370 ml of 30% unstabilized H₂O₂, 250 ml of aqueous ammonia and 9 gallons of water. They are then placed in a cascade water rinse with the final rinse being with 0.1 micron filtered water. They are then spin-dried and are ready for optical coating. An alternative to this procedure is the "RCA Clean" as described in Polymer Surfaces and Interfaces, edited by W.J. Feast and H.S. Munro, John Wiley and Sons, N.Y, N.Y, page 212, 1987. This process has been widely modified throughout the semi-conductor industry.

For glass, the degree of surface character or the irregularity is discussed in terms of glass. The diffuse reflective capability of the surface described here refers to the degree to which the reflection is scattered rather than the pure specular reflection. Diffuseness is a function of the surface topography and because the relevant topography is much larger than the interference film or biofilms, the fuzziness is not expected to vary significantly for different films. The film will, of course, affect the lightness or color of the reflected light, but not its specularity.

The surface topography, and hence fuzziness or irregularity may be characterized with a surface profilometer, such as the Dek-tak® (Sloan Technology Corp., Santa Barbara, California). The Dek-tak® provides readings on the separation or distance between surface features and an average value for the height of surface features over a defined region of a surface. One useful measure of the surface is the RMS roughness divided by the average peak spacing, where a peak is defined to be a protrusion with a height of at least 50% of the RMS roughness. Since roughness is a function of the reflectivity versus angle, it may be quantified by measuring the angle dependence of the reflectivity. For a light source incident at 30° from normal, the reflected light intensity on a photodiode should be measured as a function of the angle from 0° to 90°. The wafer selected should optimally show a smoothly varying reflectivity over the angular range viewed. Using a HeNe laser light source, the present reflectance from a roughened surface should be less than 5%, assuming a polished wafer reflects at 100%.

In a preferred embodiment of the present invention, articles comprising the non-specular, i.e. irregular, surfaces which support the layer or layers of anti-reflective material capable of being activated to attach to an analyte receptive material, are characterized in that the irregular or textured surfaces, when measured by a profilometer, measure or exhibit values between from about 2700 and about 3295 with a preferred measurement about 2995. This value represents the RMS roughness divided by the average peak of the textures and whose reflectance, as measured by a HeNe laser light source, is less than about 5%.

In addition to abrasive lapping, a wide range of chemical or plasma etching techniques are suitable for providing the diffuse light properties of the substrate. Glass can be modified to a diffuse light reflecting surface using a HF etch as in the production of frosted glass.

The third embodiment of this invention is prepared in a similar manner as either the first or the second embodiment with the exception of the affixation of the anti-reflective layers. The third embodiment is designed to be readable by an instrument such as an ellipsometer, photoreflectometer, comparison ellipsometer, etc., and not necessarily by the unaided eye; therefore the use of anti-reflective material which generated a visible interference color is optional in the third embodiment.

The instrumented device is formed by the same methods as the first and second embodiments with the exception that the substrate is not necessarily coated with anti-reflective material and the substrate can be directly activated to allow the binding of receptive material.

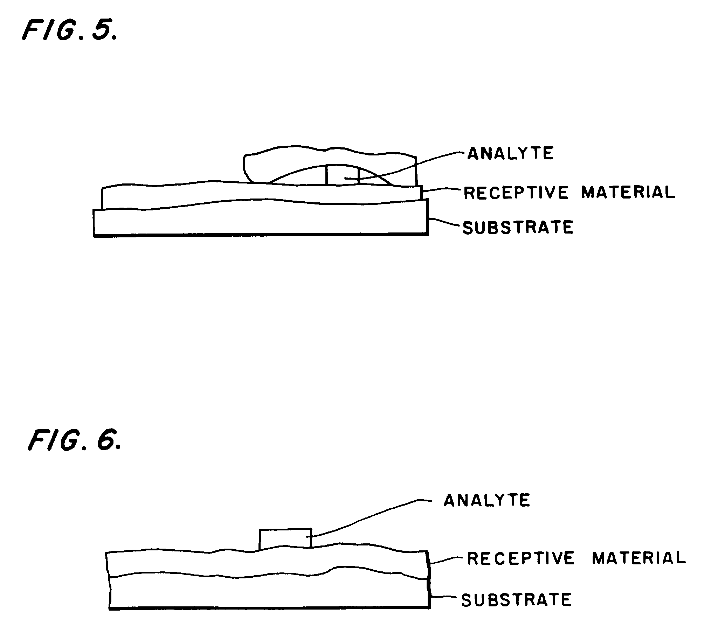

It was a highly unexpected discovery that the device when produced without anti-reflective material generated a signal which was easily detected by a variety of optical instruments. It was also discovered that when the first and second embodiments were read under an instrument often filters were necessary to achieve the results generated by the device with no anti-reflective material coated on the substrate (see Figures 5 and 6).

### STEP 2: Affixation of the Anti-reflective Layers onto the Substrate

Silicon monoxide is one example of an anti-reflective (AR) thin film that may be coated on to the substrate by standard thin film coating techniques known in the semiconductor and optical industries. The coating can be performed by electron bombardment or by evaporating the material on to the apparatus in a low temperature vacuum chamber, capable of producing a vacuum of at least 10⁻⁵ Torr or by any other equivalent method. These silicon based coatings are commercially coated, to the hereinafter mentioned specifications. Silicon wafers, glass, or other diffuse light reflecting substrates may be coated in these processes.

In this example, silicon monoxide was coated to a thickness between 450 Angstroms and 650 Angstroms, which is slightly less than a quarter of a wavelength of light in optical thickness. Alternatively, the anti-reflective material can be coated between 1800 Angstroms and 12,500 Angstroms, which is approximately the thickness of three quarters to several quarters wavelength of light in optical thickness to produce a more sensitive apparatus. The lambda is the wavelength chosen for suppression, then the reflections will be out of phase and a reflective minimum for the incident light is achieved. Optical thickness is defined as the physical thickness of the coating multiplied by the refractive index of the AR materials. Therefore, in the preferred embodiment of this invention, the optical coatings are usually deposited with a thickness of 1/4 wavelength of light in optical thickness.

To establish equal intensities the refractive indices should form a geometric progression. Since the refractive index of air is 1.0 the refractive index of the AR coating should be approximately equivalent to the square root of the substrate. The greatest reduction in reflection occurs for an anti-reflective coating having a thickness of approximately one-fourth wavelength of light in optical thickness in the medium, and a refractive index which is the square root of the product of the indices of the media directly above and below it. Thin films of silicon monoxide on the apparatus exhibit a refractive index of 1.8-2.0 which is approximately the square root of the refractive index of the silicon wafer which is 4.1. Thus, affixation of anti-reflective layers diminishes the loss of incident light due to reflection.

The number of anti-reflective layers coated onto the substrate can be any integer 1 to infinity with the preferred number of layers of material being two. The number of layers is limited only by two factors: the indices of refraction of the selected materials should follow a geometric progression, and the cost involved in production of multilayer coatings.

The slide apparatus when coated with the silicon anti-reflective layers suppresses certain wavelengths in the visible blue light range and therefore reflects a gold interference color. Although a gold interference color is utilized in this combination of elements the visual interference color of the slide can be any suitable color in the spectrum; depending on the material of the substrate selected, the chemical composition of the anti-reflective layers selected, and the thickness and number of the layers coated.

### STEP 3: Adhering Receptive Material to the Slide Apparatus

Prior to adhering the receptive material to the optically coated substrate, the surface of the apparatus must be thoroughly stripped of foreign particles, whose unwanted presence could cause non-specific binding, or background signals. To accomplish this, the apparatus is treated by an oxygen plasma etchant in a vacuum chamber. The coated apparatus is placed into a vacuum chamber, which is evacuated to 0.7 Torr. The oxygen is excited by a plate current of 175 D.C. milliamperes, 250 RF watts to form an oxygen plasma.

The oxygen plasma etching process is allowed to continue for five minutes to assure the removal of all undesired organic matter. This oxygen plasma etching process should be performed immediately prior to adhering the receptive material.

The cleaned apparatus then is chemically activated to facilitate the covalent binding of the receptive material to the uppermost anti-reflective material, silicon dioxide. Adherence of the receptive material can be done by a number of methods; such as Langmuir-Blodgett coating techniques, by adsorption, or by any other mechanism which will adequately affix the receptive material to the activated coated substrate. As previously discussed in the third embodiment the cleaned substrate is chemically activated to facilitate the adherence of the receptive material to the device.

Because this example is coated with silicon dioxide, silane chemistry is used to activate the surface and covalently bind the receptive material to the surface. Twenty-five silicon monoxide wafers are placed into a quartz rack which is inserted into a vacuum desiccator; within this desiccator a small vessel is inserted containing approximately 5 microliters of bisamino-silane. Specifically, N-(2-aminoethyl)-3-aminopropyl-trimethoxysilane, boiling point of 140 degrees at 15 Torr, is used. The boiling point of the aminosilane compound determines the optimal pressure and temperature for running the vapor phase deposition. The vacuum desiccator is evacuated to 0.06 Torr for 30 minutes. Then the temperature of the desiccator is raised to 100 degrees C over the course of one hour, to produce a coating of silane of approximately 20-30 Angstroms. This coating remains stable for at least 6 months.

The chemical activation coating can be performed by alternative methods as depicted in the examples, including, but not limited to, chemical activation by solution deposition, spin coating, or any other mechanism capable of preparing the surface for affixing the receptive material(s) to the surface of the apparatus.

The apparatus is now prepared for the attachment of the receptive material. The specific example used here is a test for the detection of rheumatoid factor, the analyte, in a body fluid. Three acceptable receptive materials or ligands, namely Bovine Gamma Globulin, human IgG, or Bovine Immunoglobulin G (IgG), have been utilized to bind the analyte. For differing analytes, different receptive material must be utilized. This example uses the most cost efficient ligand Bovine Gamma Globulin (hereinafter called BGG). The silane coated apparatus is placed into a 8 cm by 3 cm by 2 cm plastic cell culture box (any suitable container can be used). Twenty mls of 10 mM phosphate buffered .9% saline solution (hereinafter referred to as PBS) adjusted to a pH of 7.4 is combined with 200 micrograms per ml of BGG (the ratio of weight/volume gives an excess of ligand and it is subject to a wide range of useful concentrations), and placed into the plastic box. To this solution is added a ratio of 1% per volume of glutaraldehyde. The solutions in which the receptive material is mixed will depend on the characteristics of the activation step and on the choice of receptive material.

The box containing tne apparatus that is covered by the receptive material solution is incubated at room temperature in an agitation bath. The agitation enhances the uniform adherence of the receptive material to the coated apparatus. The binding of the BGG to the apparatus in this example is by the formation of covalent bonds between the activated silane functional groups and the protein molecule. The adherence of the chosen receptive material can be performed by a variety of known techniques. The preferred technique will be a function of the type of binding required and the type of receptive material employed.

Following incubation, unbound bovine gamma globulin proteins are removed from the apparatus by rinsing the apparatus thoroughly with deionized water. The preparation of the pre-formed article is completed by placing the apparatus under a stream of N₂ or pressured air, or by air drying the apparatus. However, in the preferred embodiment of this invention an optional blocking step is implemented prior to drying the slide.

Following the rinsing of the slide apparatus to decrease the amount of non-specific binding the apparatus is placed in a blocking solution and allowed to incubate for one hour in an agitated bath. The solution was made of 2 µg/ml of acid hydrolyzed casein plus 1% glycerol volume to volume and 2% sucrose weight to volume in PBS sufficient to bring the total volume to 20 mls.

Following the agitated incubation, the apparatus is rinsed with deionized water to remove all unbound blocker, and the slide is dried. Various blocking agents and blocking solutions known to those skilled in the art can be utilized for example BSA, milk, spermidine, glycine, ethylene diamine, etc.

### STEP 4: Assay for Analyte Affixed to the Apparatus

Prior to contacting the fluid containing the analyte with the pre-formed article, the fluid is diluted by adding aliquots of the fluid to phosphate buffered saline. Although dilution is not necessary it is the preferred means for lessening non-specific binding. For this example, the optimum preferred dilution for this assay is 1:1 (volume serum:volume diluent), although wide latitudes of dilution can be used, depending upon the nature of the fluid to be assayed and the assay detection technique employed. Alternative sample diluents can be utilized to prevent adventitious adherence of undesired material to the pre-formed article, for example 20 mM TRIS at a pH of 8.0.

The diluted sample of fluid suspected of containing the analyte of interest is then placed into contact with the pre-formed slide. The pre-formed article can be dipped into the diluted sample, or a small drop of the fluid can be smeared or lightly contacted with the surface of the article. Adherence of the analyte to the article is enhanced by incubation of the slide on a heating block at 45 degrees C for three minutes, or optionally the pre-formed article can be placed under a heat lamp for five minutes, or alternatively, the device can incubate at room temperature until the moisture in the sample has evaporated. It should be understood that the temperature at which the device incubates is a function of the receptive material and analyte selected. Following the incubation, the pre-formed article is fully rinsed to remove from its surface any unbound material. Then the device is dried with a stream of N₂ or pressurized air, or any equivalent means for removing the rinsing solution and preventing water spotting on the slide's surface (see Figure 2).

### STEP 5: Assay Detection Techniques

Two assay detection techniques, visual and instrumented, are used for the detection of the bound analyte. Any suitable instrumented method of detection can be employed. For the examples described hereinafter, an interference color phenomena is used to visually ascertain whether the fluid contains the analyte of interest.

The pre-formed article can be illuminated with a polychromatic or monochromatic light source to determine whether analyte has bound to the surface. The pre-formed (or unreacted) article under polychromatic light reflects a gold interference color with an absorbance maxima of approximately 476 nanometers, in comparison the reacted article under polychromatic light reflects the gold color where no analyte is bound; and it reflects a purple or a blue interference color with an absorbance maxima between 550 nanometers and 650 nanometers where the analyte is bound. The color of the reacted spot depends on the concentration of the analyte bound to the surface. The higher the concentration the more intense the color change appears.

When the analyte reacts with the receptive material, the physical thickness of the material on top of the substrate either increases or decreases. This thickness change results in a change in the optical pathway; the new optical pathway causes the interference color to change. In order to achieve the correct phase shift for destructive interference in the preferred method of this invention, the thickness of the SiO, or of any AR film coating, is slightly less than an odd number of quarter wavelengths of the incident light. Because air is a less dense medium than the AR films or the substrate, there is a pi/2 phase shift for reflections at both interfaces. The identical phase shift cancels each other. Therefore, to determine the net phase shift the only factor is the optical path difference, which is defined as 2d x n_{f}. The definition of d is the actual measurement of the AR thickness, and n_{f} is defined as the AR film's refractive index. The actual phase shift is therefore 2dn/lambda multiplied by 2pi if in radians, or 360 if in degrees. The optical thickness of the AR film is some odd quarter wavelength of the incident light; in this example the actual optical thickness is approximately equivalent to lambda/4, lambda being the wavelength selected for peak performance. Therefore, the phase shift is about 180 degrees and there is partial destructive interference which reveals a gold interference color because certain of the wavelengths in the blue region of visible light, at approximately 465-480 nanometers, are suppressed.

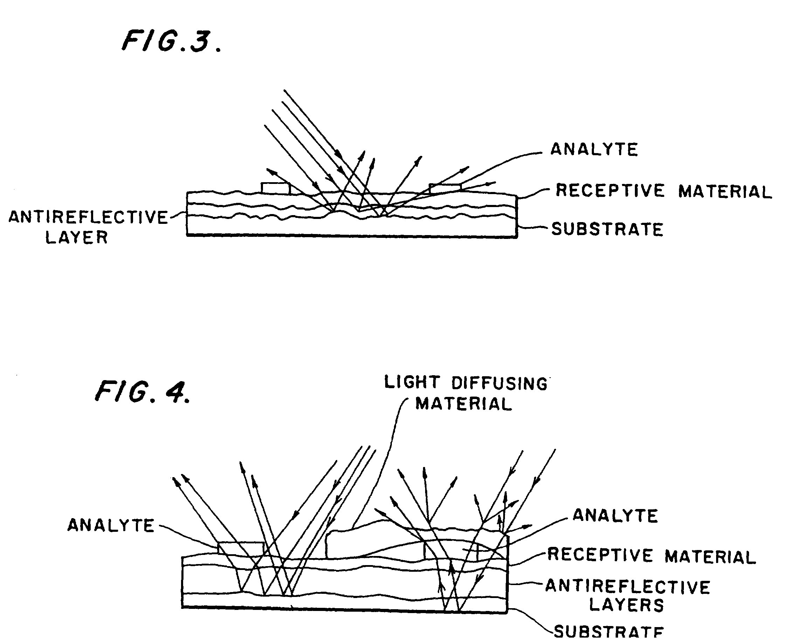

When the analyte binds to the receptive material on top of the article, the optical path is either increased or decreased. In this example, the actual thickness change incurred by the binding reaction between the rheumatoid factor and the Bovine Gamma Globulin is an increase in material of approximately 20-500 Angstroms. This change in the optical path yields a suppression of the gold wavelengths near 450 nanometers, thus resulting in a blue or purple interference color defining the area where the analyte bound. The purple color on the diffuse substrate is partially scattered, partially reflected (see Figure 3). The purple color on the smooth surfaced substrate is specularly reflected prior to addition of the light diffusing material (see Figure 4).

It is surprising that the application of an anti-reflective coating maintains its ability to produce interference effects on a diffuse or textured substrate. Anti-reflective films are assumed to be effective only when the substrate is highly reflective. They are expected to display angle dependence in the generation of interference colors. For AR films in the optical industry, these are the desired properties.

For a visual assay with diagnostic application, the reflectivity and angle dependence are serious disadvantages. The high degree of reflectivity obtained with the prior art substrates (Nygren et al, U.S. Patent 4,558,012) produce contrasting interference colors but the mirror-like surface is very distracting to the inexperienced viewer. The eye tends to focus on other images which are reflected in the substrate and can be directed away from viewing a reacted zone on the substrate. The greatest limitation of the specular reflecting substrate is the dependence of the perceived interference color on the angle of viewing. This is a severe limitation at or near the detection limits of the assay system. These substrates require a fairly sophisticated level of training to correctly interpret a weak positive response. The diffuse substrate, if properly selected, maintains the clear generation of interference color, without the distractions associated with viewing the specular surface. In addition, these substrates do not produce an interference color angle dependence for viewing. This allows the visual interpretation of very weak positives to be clearly made by inexperienced individuals. Thus, the overall sensitivity of a qualitative, visual assay is greatly improved with these substrates.

To avoid the need for signal enhancement techniques, the interference color change should be optimized to yield a contrasting interference color when reacted. This provides a color contrast to which the human eye is highly sensitive. It should be understood that any color shift whether visible to the human eye or not can be utilized as a detection signal, because the instrumented detection method is highly sensitive to nonvisible changes in thickness. The instrument frequently used to quantitatively analyze the amount of bound material is the ellipsometer, although various other instruments can perform a similar analysis. Although all three embodiments of the present invention can be read by an instrument, the third embodiment is specifically designed for instrumented detection. The instrumented detection allows the assay to be performed on a specularly or non-specularly reflecting surface with comparative results.

Ellipsometry is an optical method for determining the thickness and refractive index of extremely thin films. The technique exploits changes from planar to elliptical polarization which occur as the light beam is reflected. The shape and orientation of the ellipse are affected by the angle of incidence and properties of the reflective surface. Elliptical orientation is thus a very sensitive measure of the thickness of a film coating the reflective surface. The proper incidence angle for a comparison ellipsometer yields optimal detection of film thickness to about 3 Angstroms. This corresponds to a sensitivity of approximately 1/1000 the wavelength of the light illuminating the surface.

The Sagax® comparison ellipsometer compares the thickness of the reacted slide with a reference film that has a thickness nearly equivalent to the unreacted slide. The polarized light is reflected off both surfaces and converted into a digitized image. The image is converted into gray scale values in accordance with the light intensity. These gray scale values are a faithful representation of the actual film thickness.

Known concentrations of analyte reacted on an article were read on the ellipsometer and the resultant data were used to generate a standard curve. The concentration of the analyte, rheumatoid factor, in this sample is calculated in relation to the previously generated standard curve. It should be apparent that other methods of measurement of the analyte concentration can be employed within the scope of the present invention, such as reflectometry, colorimetry, profilometry, and the like.

In the second embodiment of this invention, the regular surfaced substrate was used. To produce the less angle dependent diffuse reflection the article is covered with a light diffusing material prior to the visual or instrumented detection of the analyte. This material can be permanently coated or affixed onto the article or alternatively it can be removable. An opaque non-glare piece of glass approximately four inches in diameter and 1/16th of an inch in thickness was placed over the second embodiment to produce a diffuse reflection or an attenuated reflection. Materials such as opaque plastics, smoked glass, or any other functional equivalent material, can be used to produce a diffuse reflection an attenuated reflection, or a light scattering effect.

### EXAMPLE 1

### Detection of Rheumatoid Factor Using Thick Anti-Reflective Coatings

A silicon wafer, approximately four inches in diameter and .02 inches in thickness having on one side a highly polished surface and on the other a diffusely reflecting surface, was coated with a combination of silicon monoxide and silicon dioxide to a thickness of 11,893 Angstroms of anti-reflective material so that both sides received an AR coating. This wafer was cut in half and one sample assay was performed on the diffusely reflecting surface, the second sample assay was performed on the smooth surface. This coated wafer was activated by application of N-(2-aminoethyl)-3-aminopropyl trimethoxysilane by the following procedure:
1. The coated wafer was oxygen plasma etched for five minutes in a vacuum at 0.7 Torr, with an oxygen atmosphere and a plate current of 175 D.C. milliamperes and 250 RF watts.
2. Immediately upon removal, the coated wafers were placed in a quartz rack and inserted into a vacuum desiccator with a vessel containing 5 microliters of N-(2-aminoethyl)-3-aminopropyltrimethoxysilane. The vacuum was run at 0.06 Torr for 30 minutes. Then the temperature of the desiccator was raised to 100 degrees over the course of one hour to complete the vapor phase deposition of aminosilane.
3. 200 micrograms/per ml of BGG and 20 mls of PBS (previously described) and 1% by volume of glutaraldehyde were combined to form the receptive material solution. The wafers were placed in a petri dish and the receptive material solution was added.
4. To enhance the adherence of the receptive material the wafers were allowed to incubate at room temperature in an agitation bath for 15 hours.
5. Following incubation the unbound bovine gamma globulin proteins were removed from the wafers, by rinsing with deionized water.
6. Following the rinsing step the wafers were placed in the previously described casein blocking solution to decrease non-specific binding. The petri dish containing the wafers and the blocking solution were incubated at room temperature in an agitating bath for one hour.
7. Following the blocking process all excess blocker was removed by rinsing with deionized water. Then the slide was dried under a stream of nitrogen. The pre-formed wafers were then used to determine the presence of rheumatoid factor in two positive serum samples. A smooth surface assay and an irregular surface assay as described hereinafter, using the coated wafer prepared as previously described, generated the data in Table 1 by the following procedures:
a. Each serum sample was diluted by adding PBS (previously described) at a 1:1 ratio (volume serum: volume diluent).
b. 5 microliters of each diluted sample were placed on each wafer; and the wafers were allowed to incubate on a heating block at 45 degrees C for three minutes to enhance analyte adherence.
c. The wafers were then rinsed with deionized water to remove all unbound material, then dried with a stream of pressurized air.

Upon visual inspection of the unreacted portion of the diffuse wafer under polychromatic light, the wafer reflected a diffuse green color at most viewing angles. Although at more oblique angles a diffuse rose color appeared. The two positive sera samples reflected a diffuse rose colored spot which was visible at most viewing angles, but shifted to a green colored spot at more oblique angles. When the diffusely reflecting wafer was placed under a green monochromatic light with a wavelength of 546 nanometers the unreacted surface diffusely reflected a green color and the reacted portion reflected a black spot at most viewing angles. Similar inspection of the unreacted, specularly reflecting wafer under polychromatic light revealed a green specular reflection at normal to 30 degrees off normal viewing angles and at angles greater than 30 degrees the wafer appeared rose colored. Dependent on the angle of incident light the reacted spot was visible, although visualizing a weak positive was very difficult. The smooth wafer was then covered by a light diffusing plastic sheet approximately 0.01 inches in thickness which made the reacted spot more easily distinguishable from the background color. The plastic also allowed the spots to be visible at angles of incident light greater than 30 degrees from normal.

**TABLE 1**

| **Thickness Change of Reacted Wafers Due to the Binding of Rheumatoid Factor** | | | |
|---|---|---|---|
| Diffusely Reflecting Wafer | Reacted Wafer Thickness | Wafer Thickness | Thickness Change |
| Positive Sample 1 | 12,003 A | 11,893 A | 110 A |
| Positive Sample 2 | 12,032 A | 11,893 A | 139 A |
| | | | |

| Specularly Reflecting Wafer Covered With Light Diffusing Material | Reacted Wafer Thickness | Wafer Thickness | Thickness Change |
|---|---|---|---|
| Positive Sample 1 | 11,999 A | 11,890 A | 109 A |
| Positive Sample 2 | 12,031 A | 11,890 A | 141 A |
| | | | |

Thickness changes measured in Angstroms.

### EXAMPLE 2

### Detection of IgE Antibodies Specific for Birch Pollen

A silicon wafer four inches in diameter and 0.02 inches in thickness, with a smooth surface and a diffusely reflecting surface was coated on both sides with two anti-reflective layers, one of silicon monoxide at approximately 500 Angstroms thick and a second of silicon dioxide to a thickness of 50 Angstroms. The diffusely reflecting coated surface of the wafer measured 531 Angstroms prior to the activation step. The wafer was activated by application of diluted purified nitrocellulose by the following procedure:
1. The wafer was placed on a photoresist spin coater and washed with 3 mls of acetone to remove foreign particles.
2. 300 microliters of the hereinafter described solution was then sprayed onto the spinning wafer. The solution consisted of 0.0829 grams of purified nitrocellulose (available from Balzer's Union) dissolved in four mls of pentyl acetate.
3. The activated wafer was then removed from the spin coater and heated in an oven at 120 degrees C for one hour. The heating process aided in eliminating any excess pentyl acetate, and aided the adsorption of the nitrocellulose to the anti-reflective material. The activated substrate was measured on an ellipsometer to be 552 Angstroms.
4. The receptive material, birch pollen extract, was made by dissolving 1/2 gram of birch pollen (commercially available) into 5 mls of a 10% isotonic phosphate buffer comprised of 2.2 grams of dibasic potassium phosphate with 0.1% Tween by volume in 1000 mls of deionized water adjusted to a 7.5 pH.
5. The birch pollen buffered solution was stirred for 3 hours to extract the surface allergens. After being stirred, the solution was centrifuged for 20 minutes at 1500 RPM, after centrifugation, the supernate liquid containing the birch pollen extract was decanted off and stored.
6. The 1/2 ml of the birch pollen extract was diluted in 20 mls of PBS and the wafer was submerged in this solution. The dish was then agitated overnight at room temperature to facilitate the adsorption of the receptive material to the nitrocellulose activation material. The blue colored wafer was measured and a 39 Angstrom increase was recorded.

The pre-formed wafer was then used to determine the presence of IgE antibodies in four serum samples from individuals with:
1. Acute allergic response (Acute)
2. Strong allergic response (Strong)
3. Mild allergic response (Mild)
4. No apparent response (Normal)

An assay as described hereinafter, using the coated wafer, prepared as previously described, generated the data in Table 2 by the following procedures:
1. 5 microliters of each serum sample were placed on the surface of the wafer. The wafer was then incubated at 37 degrees C for 15 minutes to evaporate all moisture from the sample, and to facilitate the adherence of the IgE antibodies specific for birch pollen.
2. The wafer was rinsed briefly in deionized water to remove all unbound material. Then the wafer was placed under a stream of N₂ to dry the surface. Upon visual inspection of the wafer under polychromatic light, three of the serum samples showed diffusely reflecting white spots against the bright blue background, the normal sample showed no visual color change. The intensity of the white spot correlated well with the level of allergic response. The acute sample was an extremely bright spot, the high sample was slightly less distinctive and the mild spot was readily visible but less distinctive than the other two samples.
3. The change in the thickness of the reacted portion of the wafer was measured in Angstroms on an ellipsometer.

**TABLE 2**

| **Thickness Change due to IgE Antibody Adherence** | | | |
|---|---|---|---|
| | Concentration of IgE in Sera | Total Angstrom Thickness | Increase Due To Analyte |
| Acute | 3.5 ng/ml | 1230 A | 109 A |
| Strong | 0.7 ng/ml | 1179 A | 58 A |
| Mild | 0.35 ng/ml | 1153 A | 32 A |
| Normal | 0 ng/ml | 1135 A | 14 A* |

| | | | |
|---|---|---|---|
| *Due to non-specific binding. | | | |

### EXAMPLE 3

### Detection of Carcinoembryonic Antigen (CEA)

A silicon wafer with a highly polished side and a diffusely reflecting side was coated on both sides with an anti-reflective material, silicon oxynitride, to a thickness of between 500 -650 Angstroms of material by Meadowlark Optics. The irregular side surface was chemically activated by application of N-(2-aminoethyl)-3-aminopropyl trimethoxysilane by the following procedure:
1. The coated wafer was oxygen plasma etched for 5 minutes in a .71 Torr oxygen atmosphere with a plate current of 175 D.C. milliamperes and 250 RF watts.
2. Immediately upon removal from the oxygen plasma etcher the wafer was placed in a quartz rack which was inserted into a vacuum desiccator with an attached vessel into which 5 microliters of the aminosilane was placed.
3. The vacuum desiccator was run at .06 Torr for 30 minutes. Then the temperature of the desiccator was raised to 100 degrees C over the course of one hour. This process activated the wafer by the vapor deposition of the aminosilane onto the water surface.
4. The wafer was now prepared for the adherence of the receptive materials. First, a solution comprised of 20 ml PBS, .1% glutaraldehyde by volume, and 150 microliters of IGAP which is a synthetic polypeptide that covers the active region of protein A was made up. (Other experiments substituted protein A or protein G for the IGAP and similar results were achieved.)
5. 200 microliters of monoclonal antibody (polyclonal can be substituted) specific for carcinoembryonic antigen was pipetted into the IGAP solution at 10 µg/per ml.
6. The combined solution was placed on top of the wafer in a petri dish and was incubated at room temperature in a shaker bath for two hours. When removed the petri dish was transferred to a cooling unit and allowed to incubate for 48 hours at 4 degrees C to further the adherence of the receptive antibody material. To remove all unbound material the wafer was rinsed with deionized water and dried in a stream of N₂.

The pre-formed wafer which diffusely reflected a tan color was then used to determine the presence of CEA antigen in five serum samples with known CEA concentrations.

An assay as described hereinafter, using the coated wafer, prepared as just described, yielded the data in Table 3 and the data in Chart 1 by the following procedures:
1. 10 microliters of all five samples of serum were contacted with the surface of the wafer, and allowed to incubate at 37 degrees C for seven minutes.
2. After seven minutes the wafer was rinsed in deionized water to remove all unbound material. Then the wafer was dried under a stream of nitrogen. Visual inspection of the slide revealed small pinkish spots with color intensities which correlated with the increasing analyte concentration levels.
3. The change in the thickness of the reacted portion of the wafer was measured in gray scale units by a Sagaxe comparison ellipsometer.

**TABLE 3**

| **Measurement of Bound CEA Antigen** | | | | |
|---|---|---|---|---|
| CONCENTRATION (ng/ml) | Average Gray Scale | Standard Deviation | CV | +/- 2 SD |
| 0.0 | 41.0 | 2.68 | 6.6% | 35.6-46.4 |
| 0.5 | 48.3 | 2.95 | 6.1% | 42.4-54.2 |
| 0.9 | 51.7 | 2.59 | 5.0% | 46.5-56.9 |
| 3.7 | 65.1 | 7.02 | 10.8% | 51.1-79.1 |
| 7.5 | 98.8 | 3.79 | 3.8% | 91.2-106.4 |

### EXAMPLE 4

### Detection of Group A Streptococcus

Three silicon wafers with highly polished surfaces, and three silicon wafers with diffusely reflecting surfaces each having a refractive index of approximately 4 to 4.08 at a specified wavelength, were used in this test. Group A was coated with approximately 550 Angstroms of silicon monoxide having an index of refraction of 1.97 at a specified wavelength. Group B was coated with approximately 560 Angstroms of silicon oxynitride having an index of refraction at a specified wavelength of 1.95. Group C was coated with approximately 550 Angstroms of boron oxide having an index of refraction at a specified wavelength of 1.9. These coatings closely approximate the equation used for coating one optical layer to the surface of the substrate.

The receptive material was coated to a thickness of 25 to 30 Angstroms. Three groups of wafers were chemically activated to allow attachment of the receptive material. The activation was by application of N-2(2-aminoethyl)-3-aminopropyltrimethoxysilane by the following procedure:
1. The coated wafers were oxygen plasma etched for 5 minutes in a 0.70 Torr oxygen atmosphere with a plate current of 175 D.C. milliamperes and 250 RF watts.
2. Immediately upon removal from the oxygen plasma etcher the wafers were placed in a quartz rack which was inserted into a vacuum desiccator with an attached vessel into which 2.5 microliters of the aminosilane was placed.
3. The vacuum desiccator was evacuated to 0.06 Torr for 30 minutes. Then the temperature of the desiccator was raised to 100 degrees C over the course of one hour. This process activated the wafer by the vapor deposition of aminosilane onto the wafers' surfaces.
4. The wafers were now prepared for the adherence of the receptive materials. First, 250 micrograms per milliliter of Bovine Serum Albumin (BSA) was dissolved in Phosphate Buffered Saline (PBS). 10 milliliters of this solution was adjusted to pH 8.5 and was pipetted into a plastic culture cell box containing the six wafers.
5. 50 microliters of a 25% glutaraldehyde in water solution was added to the culture cell box. 25 microliters of a 50 ml micrograms per ml of a Protein A solution was also added to the culture cell box.
6. The six wafers in the culture cell box were incubated in a shaker bath at room temperature for one and a half hours.
7. 200 microliters of a commercially available (from Ventrex, Inc.) anti-Strep A (raised in rabbits) was pipetted into the culture cell box; and allowed to incubate for another hour in the shaker bath at room temperature.
8. Following incubation the culture cell box was refrigerated at 4 degrees C for approximately 48 hours to further adherence of the receptive antibody material to the device. To remove all unbound material the wafers were rinsed with deionized water and dried in a stream of N₂.

The six wafers appeared shades of gold with three of the wafers providing a specular reflection and three of the wafers providing a non-specular diffuse reflection. These wafers were then used to determine the presence of Strep A in either an intact bacterial form or as a bacterial lysate.

An assay technique is described hereinafter:
1. Each wafer was separated and placed into a small plastic test kit. The test kit was designed with two rough grade filter paper pads attached to the inside of the top cover. Each test kit received seven microliters of a positive control placed on one pad and seven microliters of a negative control placed on the second pad. The positive control was heat killed Group A streptococcus in a buffered solution with a .02% sodium azide. The negative control was taken from a solution of 50 micrograms of BSA per ml of PBS.
2. The cover of the test kits were closed placing the filter pads in contact with the coated wafer for one minute.
3. The covers were opened and the wafers were allowed to air dry for one minute. Then the wafers were rinsed with distilled deionized water and dried with pressurized air.

Visual inspection of the wafers revealed highly visible purple spots on the non-specular wafers and visible purple spots on the specular wafers. Visual inspection of the non-specular wafers demonstrated a lower angle dependence than that of the specular wafers. The reacted colors became less vibrant at lower concentrations, although all spots were clearly distinguishable. An instrument was used to verify the increase of mass produced by the binding reaction on the surface of the wafer. The change in mass or thickness was measured in gray scale units by a Sagax® Comparison Ellipsometer.

The foregoing examples serve to illustrate the efficiency and utility of this technology to detect a variety of analytes using the pre-formed slide consisting of a substrate, anti-reflective material(s), activation, and receptive material(s) to generate an interference color change as a signal of analyte attachment.

Without being bound to the substrate formats or materials utilized in the preceding examples, it is possible to utilize a diversity of combinations of substrate formats and substrate materials which are functionally equivalent substitutes capable of having anti-reflective material bound to their surface, or as described in the third embodiment are capable of being activated to allow attachment of the receptive material (see Figure 5).

The anti-reflective material(s) provides a layer which can be activated to function as the receptive material or to attach or adhere, by whatever mechanism the receptive material to the apparatus. Furthermore, the anti-reflective material utilized in the preferred embodiment of this invention is coated to be a quarter wavelength optical thickness of a designated wavelength of light to achieve the highest level of destructive light interference. The AR coating can vary from a quarter of the designated wavelength namely, because destructive interference need not be total to achieve an adequate interference color. The actual amount of AR material coated to the surface can vary widely because the designated wavelength can be almost any wavelength.

### EXAMPLE 5

### Comparison of Diffuse Surfaces with a Strep Group A Antigen Detection Assay

Wafers lapped with varying particle sizes were coated with silicon nitride to a thickness and refractive index of 500 Angstroms and n-2.0. This produces a gold interference color. The wafers were initially inspected for amount of reflectivity observed, or for the remaining specular characteristics. These wafers were then coated with aminosilane as in previous examples and then antibody coated with an anti-Strep A polyclonal antibody. The wafers were then reacted with samples containing varying levels of Strep Group A antigen and a secondary reagent in buffer, applied to the surface of these wafers and incubated for 2 minutes at room temperature. The slides were rinsed with deionized water and dried under a stream of nitrogen. No difference was observed in the amount of silane incorporated or antibody attached.

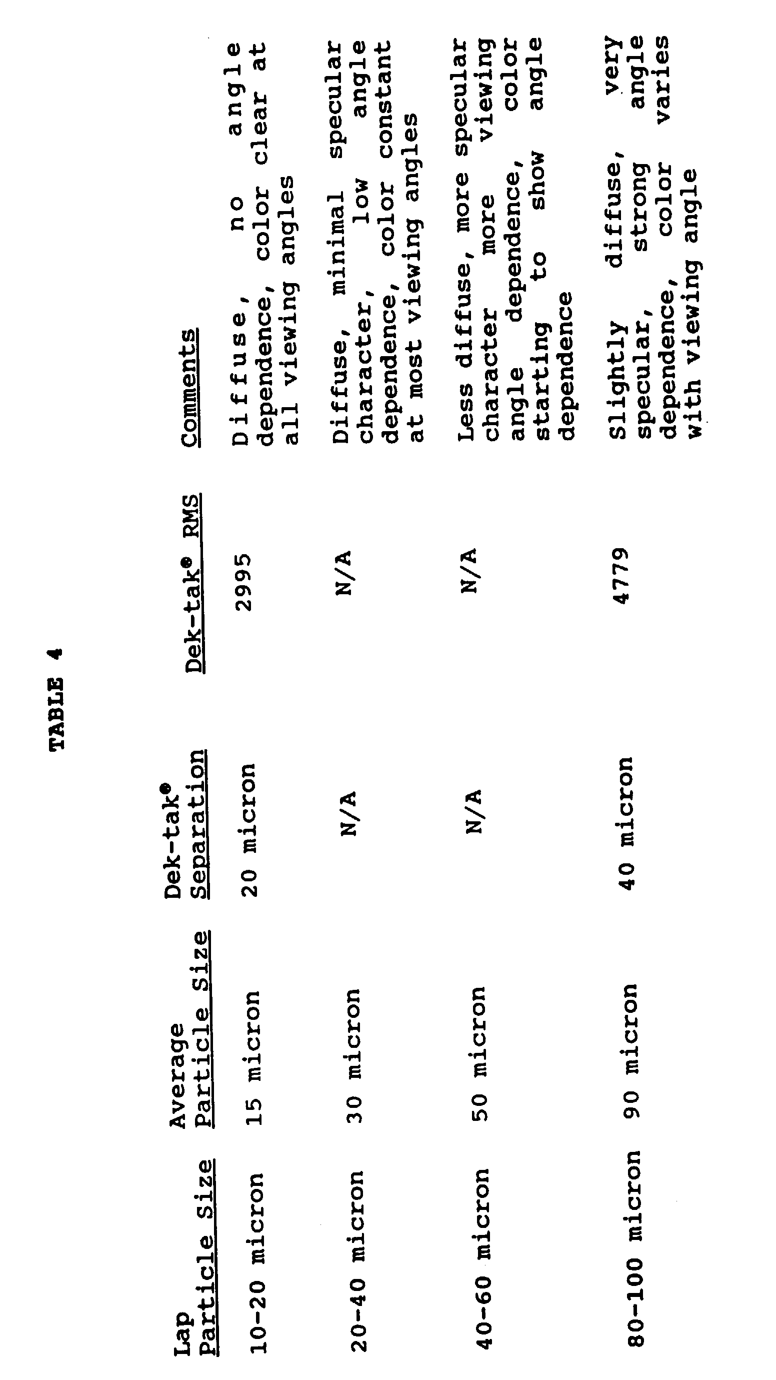

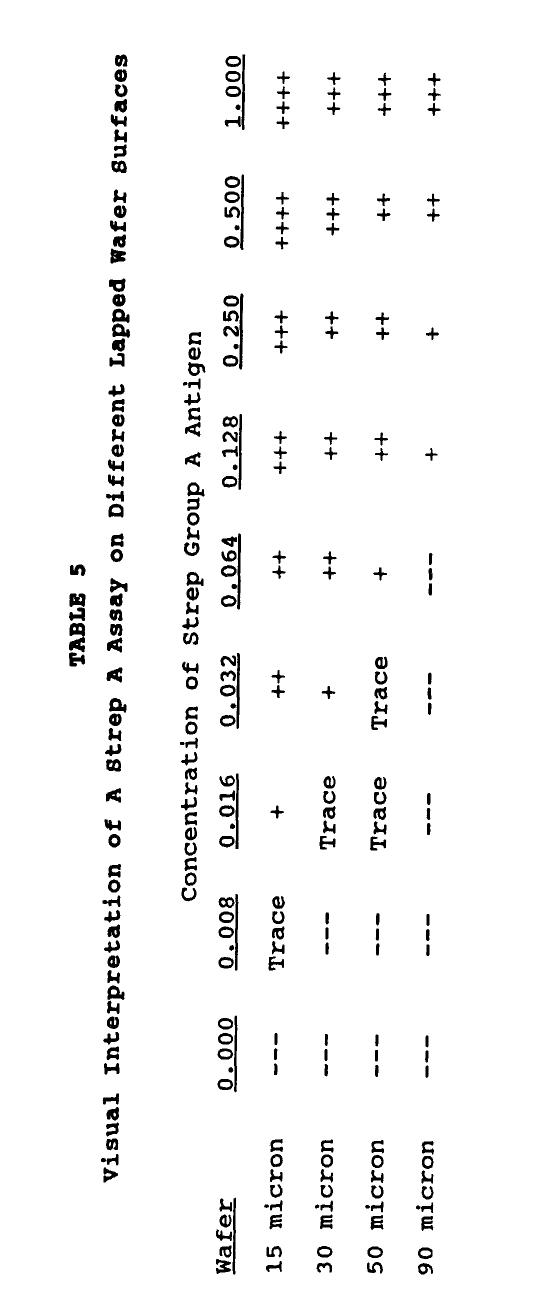

The material composition of the AR layers is selected based on the refractive index of the AR materials and the substrate materials selected; therefore almost any AR material can be utilized if the correct combination of materials is selected.

The uppermost AR material is selected to be capable of being activated to receive the receptive material. Most AR materials can be activated by some means to allow attachment of receptive material to the apparatus; therefore a multitude of materials is capable of being utilized as anti-reflective materials. Various methods of chemical activation can be utilized dependent on the composition of the AR material and the receptive material. A variety of materials, functional groups, etc. can function as the activation material or in the activation process.

The treatment of the substrate, which can be any of a variety of shapes, i.e. test tubes, wafers, glass slides, microwells, etc., and formats, i.e. a solid support, a flexible support, a gel, a pellicle and made of various suitable materials i.e., glass, silicon, plastics, such as polystyrene and the like and/or comprises a coating thereof on another support material etc., with the aforementioned technology affords many useful approaches to the detection of the analyte. This analyte detection need not be limited to visual detection of an interference color change. The color change can be in the infrared or the ultra-violet regions wherein the analyte detection is pursuant to an instrumented detection apparatus. The reacted apparatus can be qualitatively or quantitatively analyzed by an ellipsometer, or any other instrument capable of detecting by whatever mechanism the analyte binding.

The diffusely reflecting surface can be produced by using an irregular surfaced substrate, or by placing a suitable light diffusing or light interfering component or material over the top of the apparatus so that at least some of the layer capable of emitting a non-specular reflection is impinged upon, or by coating the substrate with material capable of producing a diffusely reflecting surface, or by any other suitable means for producing a diffuse reflection, an attenuated or less than total reflection, or any other non-specific reflection.

## Claims

1. Device for detecting the presence of an analyte of interest comprising:
a substrate supporting or having attached thereto, a layer of an analyte-receptive material, constituting in combination with an anti-reflective coating an optically active surface exhibiting a first interference color in response to light impinging thereon, and exhibiting a second interference color comprising a combination of wavelengths of light different from said first interference color or comprising an intensity of at least one said wavelength of light different from said first interference color, in response to said light when said analyte is present on said optically active surface;
wherein said substrate has a non-specular surface or wherein a transparent layer having a non-specular surface is provided for viewing of said optically active surface.

2. The device of claim 1, wherein said substrate comprises an optical film.

3. The device of claim 1, wherein said substrate comprises a material selected from the group consisting of monocrystalline silicon, glass, ceramic, polycrystalline silicon, metal, plastic, and composites of these materials.

4. The device of claim 1, wherein said anti-reflective coating comprises a material selected from the group consisting of: silicon dioxide, silicon monoxide, diamond, and silicon carbide.

5. The device of claim 1 or 2, wherein said substrate is coated with a non-specular material.

6. The device of claim 1 or 2, wherein said analyte is selected from the group consisting of rheumatoid factor, IgE antibodies, carcinoembryonic antigen, an analyte specific for an autoimmune disease, an allergen, an infectious microorganism or *Streptococcus* Group A antigen.

7. The device of claim 1 or 2, wherein said non-specular surface has a reading of between 2700 and 3295 with a profilometer, wherein said value represents the RMS roughness divided by the average peak of the textures, and whose specular reflectance measured by an HeNe laser light source is less than about 5%.

8. The device of claim 1 or 2, wherein the analyte receptive layer comprises a specific binding partner for said analyte.

9. The device of claim 8, wherein said receptor layer is formed from material selected from the group consisting of antigens, antibodies, oligonucleotides, enzymes, bacteria, and nucleic acids.

10. The device of claim 1 or 2, wherein said analyte is selected from the group consisting of an antibody, antigen, enzymes, and nucleic acids.

11. The device of claim 1 or 2, wherein the analyte of interest is the Strep Group A antigen.

12. The device of claim 1 or 2, wherein said substrate comprises an additional layer, suitable for the attachment of the receptive material.

13. The device of claim 1 or 2, wherein the substrate is a light reflective material.

14. The device of claim 1 or 2, wherein the substrate is a light transmissive material.

15. The device of claim 1 or 2, wherein the impinging light is monochromatic light, or polychromatic light.

16. The device of claim 1 or 2, adapted for use with a reflectometer.

17. The device of claim 1 or 2, adapted for use with a comparison ellipsometer.

18. The device of claim 1 or 2, wherein the substrate is a frosted glass.

19. Method for detecting the presence or amount of an analyte of interest in a sample, comprising the steps of:
providing a substrate supporting or having attached thereto, a layer of an analyte-receptive material, constituting in combination with an anti-reflective coating an optically active surface exhibiting a first interference color in response to light impinging thereon, and exhibiting a second interference color comprising a combination of wavelengths of light different from said first interference color or comprising an intensity of at least one said wavelength of light different from said first interference color, in response to said light when said analyte is present on said optically active surface;
wherein said substrate has a non-specular surface or wherein a transparent layer having a non-specular surface is provided for viewing of said optically active surface;
and contacting said optically active surface with a sample potentially comprising said analyte of interest under conditions in which said analyte can interact with said optically active surface to cause said optically active surface to exhibit said second interference color when said analyte is present.

## Patentansprüche

1. Vorrichtung zum Nachweis der Gegenwart eines Analyten von Interesse, welches umfaßt:
Substrat, das eine Schicht eines Analyt-rezeptiven Materials trägt oder in Anheftung daran aufweist, welches in Kombination mit einem anti-reflektierenden Überzug eine optisch aktive Oberfläche bildet, welche eine erste Interferenzfarbe in Antwort auf darauf auftreffendes Licht zeigt, und eine zweite Interferenzfarbe, welche eine Kombination von Lichtwellenlängen umfaßt, die von der ersten Interferenzfarbe verschieden sind, oder eine Intensität mindestens einer der Lichtwellenlängen umfaßt, die von der ersten Interferenzfarbe verschieden ist, in Antwort auf das Licht zeigt, wenn der Analyt auf der optisch aktiven Oberfläche vorhanden ist;
worin das Substrat eine nicht-spiegelnde Oberfläche besitzt, oder worin eine transparente Schicht mit einer nicht-spiegelnden Oberfläche zur Betrachtung der optisch aktiven Oberläche vorgesehen ist.

2. Vorrichtung von Anspruch 1, worin das Substrat einen optischen Film umfaßt.

3. Vorrichtung von Anspruch 1, worin das Substrat ein Material umfaßt, das aus der aus monokristallinem Silicium, Glas, Keramik, polykristallinem Silicium, Metall, Kunststoff und Verbundsstoffen dieser Materialien bestehenden Gruppe gewählt ist.

4. Vorrichtung von Anspruch 1, worin der anti-reflektierende Überzug ein Material umfaßt, das aus der aus folgendem bestehenden Gruppe gewählt ist: Siliciumdioxid, Siliciummonoxid, Diamant und Siliciumcarbid.

5. Vorrichtung von Anspruch 1 oder 2, worin das Substrat mit einem nicht-spiegelnden Material überzogen ist.

6. Vorrichtung von Anspruch 1 oder 2, worin der Analyt aus der aus Rheumatoid-Faktor, IgE-Antikörpern, karzinoembryonischem Antigen, einem für eine Autoimmunkrankheit spezifischen Analyten, einem Allergen, einem infektiösen Mikroorganismus oder dem *Streptococcus-*Gruppe A-Antigen bestehenden Gruppe gewählt ist.

7. Vorrichtung von Anspruch 1 oder 2, worin die nicht-spiegelnde Oberfläche einen Skalenwert zwischen 2700 und 3295 auf einem Profiltastschnittgerät aufweist, wobei der Wert die RMS-Rauhigkeit geteilt durch den Durchschnittspeak der Strukturen repräsentiert, und deren mittels einer HeNe-Laserlichtquelle gemessene Remission geringer als etwa 5 % ist.

8. Vorrichtung von Anspruch 1 oder 2, worin die Analyt-rezeptive Schicht einen spezifischen Bindungspartner für den Analyten umfaßt.

9. Vorrichtung von Anspruch 8, worin die Rezeptorschicht aus einem Material gebildet ist, das aus der aus Antigenen, Antikörpern, Oligonucleotiden, Enzymen, Bakterien und Nucleinsäuren bestehenden Gruppe gewählt ist.

10. Vorrichtung von Anspruch 1 oder 2, worin der Analyt aus der aus einem Antikörper, Antigen, Enzymen und Nucleinsäuren bestehenden Gruppe gewählt ist.

11. Vorrichtung von Anspruch 1 oder 2, worin der Analyt von Interesse das Strep-Gruppe A-Antigen ist.

12. Vorrichtung von Anspruch 1 oder 2, worin das Substrat eine zusätzliche Schicht umfaßt, die für die Anheftung des rezeptiven Materials geeignet ist.

13. Vorrichtung von Anspruch 1 oder 2, worin das Substrat ein lichtreflektierendes Material ist.

14. Vorrichtung von Anspruch 1 oder 2, worin das Substrat ein lichtdurchlässiges Material ist.

15. Vorrichtung von Anspruch 1 oder 2, worin das auftreffende Licht monochromatisches Licht oder polychromatisches Licht ist

16. Vorrichtung von Anspruch 1 oder 2, ausgelegt für die Verwendung mit einem Reflektometer.

17. Vorrichtung von Anspruch 1 oder 2, ausgelegt für die Verwendung mit einem Vergleichs-Ellipsometer.

18. Vorrichtung von Anspruch 1 oder 2, worin das Substrat ein mattiertes Glas ist.

19. Verfahren zum Nachweis der Gegenwart oder der Menge eines Analyten von Interesse in einer Probe, welches folgende Schritte umfaßt:
Vorsehen eines Substrats, das eine Schicht eines Analyten-rezeptiven Materials trägt oder in Anheftung daran aufweist, welches in Kombination mit einem anti-reflektierenden Überzug eine optisch aktive Oberfläche bildet, welche eine erste Interferenzfarbe in Antwort auf darauf auftreffendes Licht zeigt, und eine zweite Interferenzfarbe, welche eine Kombination von Lichtwellenlängen umfaßt, die von der ersten Interferenzfarbe verschieden sind, oder eine Intensität mindestens einer der Lichtwellenlängen umfaßt, die von der ersten Interferenzfarbe verschieden ist, in Antwort auf das Licht zeigt, wenn der Analyt auf der optisch aktiven Oberfläche vorhanden ist;
worin das Substrat eine nicht-spiegelnde Oberfläche besitzt, oder worin eine transparente Schicht mit einer nicht-spiegelnden Oberfläche zur Betrachtung der optisch aktiven Oberfläche vorgesehen ist;
und Kontaktieren der optisch aktiven Oberfläche mit einer Probe, welche den Analyten von Interesse möglicherweise umfaßt, unter Bedingungen, bei denen der Analyt mit der optisch aktiven Oberfläche wechselwirken kann, um die optisch aktive Oberfläche dazu zu veranlassen, die zweite Interferenzfarbe zu zeigen, wenn der Analyt vorhanden ist.

## Revendications

1. Dispositif pour détecter la présence d'un analyte auquel on s'intéresse, comportant :
un substrat supportant, ou comportant, fixé à lui, une couche d'un matériau récepteur d'analyte, constituant en combinaison avec un revêtement anti-réfléchissant et une surface optiquement active présentant une première couleur d'interférence en réponse à la lumière la frappant, et présentant une deuxième couleur d'interférence comprenant une combinaison de longueurs d'onde de lumière différentes de ladite première couleur d'interférence ou comprenant une intensité d'au moins l'une desdites longueurs d'onde de lumière différentes de ladite première couleur d'interférence, en réponse à ladite lumière, lorsque ledit analyte est présent sur ladite surface optiquement active ;
dans lequel ledit substrat a une surface non spéculaire, ou dans lequel une couche transparente ayant une surface non spéculaire est présente pour la visualisation de ladite surface optiquement active.

2. Dispositif selon la revendication 1, dans lequel ledit substrat comprend un film optique.

3. Dispositif selon la revendication 1, dans lequel ledit substrat comprend un matériau sélectionné parmi le groupe comprenant le silicium monocristallin, le verre, la céramique, le silicium polycristallin, les métaux, les matières plastiques, et des composites de ces matériaux.

4. Dispositif selon la revendication 1, dans lequel ledit revêtement anti-réfléchissant comprend un matériau sélectionné parmi le groupe comprenant le dioxyde de silicium, le monoxyde de silicium, le diamant, et le carbure de silicium.

5. Dispositif selon la revendication 1 ou la revendication 2, dans lequel ledit substrat est revêtu d'un matériau non spéculaire.

6. Dispositif selon la revendication 1 ou la revendication 2, dans lequel ledit analyte est sélectionné parmi le groupe comprenant un facteur rhumatoïde, des anticorps IgE, un antigène carcinoembryonaire, un analyte spécifique d'une maladie auto-immune, un allergène, un micro-organisme infectieux, ou un antigène de *Streptococcus* groupe A.

7. Dispositif selon la revendication 1 ou la revendication 2, dans lequel ladite surface non spéculaire a une lecture comprise entre 2700 et 3295 avec un profilomètre, ladite valeur représentant la rugosité quadratique divisée par le pic moyen des textures, et dont le facteur de réflexion spéculaire mesuré par une source de lumière à laser HeNe est inférieur à environ 5 %.

8. Dispositif selon la revendication 1 ou la revendication 2, dans lequel la couche réceptrice d'analyte comprend un partenaire de liaison spécifique pour ledit analyte.

9. Dispositif selon la revendication 8, dans lequel ladite couche réceptrice est formée en un matériau sélectionné parmi le groupe comprenant des antigènes, des anticorps, des oligonucléotides, des enzymes, des bactéries, et des acides nucléiques.

10. Dispositif selon la revendication 1 ou la revendication 2, dans lequel ledit analyte est sélectionné parmi le groupe comprenant un anticorps, un antigène, des enzymes, et des acides nucléiques.

11. Dispositif selon la revendication 1 ou la revendication 2, dans lequel l'analyte auquel on s'intéresse est un antigène de *Strep* groupe A.

12. Dispositif selon la revendication 1 ou la revendication 2, dans lequel ledit substrat comprend une couche additionnelle, appropriée pour la fixation du matériau récepteur.

13. Dispositif selon la revendication 1 ou la revendication 2, dans lequel le substrat est un matériau réfléchissant la lumière.

14. Dispositif selon la revendication 1 ou la revendication 2, dans lequel le substrat est un matériau transmettant la lumière.

15. Dispositif selon la revendication 1 ou la revendication 2, dans lequel la lumière d'impact est une lumière monochromatique ou une lumière polychromatique.

16. Dispositif selon la revendication 1 ou la revendication 2, adapté pour être utilisé avec un réflectomètre.

17. Dispositif selon la revendication 1 ou la revendication 2, adapté pour être utilisé avec un ellipsomètre à comparaison.

18. Dispositif selon la revendication 1 ou la revendication 2, dans lequel le substrat est un verre dépoli.

19. Procédé pour détecter la présence ou la quantité d'un analyte auquel on s'intéresse dans un échantillon, comprenant les étapes suivantes :
la fourniture d'un substrat supportant, ou comportant, fixée sur celui-ci, une couche d'un matériau récepteur d'analyte, constituant, en combinaison avec un revêtement anti-réfléchissant, une surface optiquement active présentant une première couleur d'interférence en réponse à la lumière qui la frappe, et présentant une deuxième couleur d'interférence comprenant une combinaison de longueurs d'onde de lumière différentes de ladite première couleur d'interférence, ou comprenant une intensité d'au moins l'une desdites longueurs d'onde de lumière différentes de ladite première couleur d'interférence, en réponse à ladite lumière lorsque ledit analyte est présent sur ladite surface optiquement active ;
dans lequel ledit substrat a une surface non spéculaire ou dans lequel une couche transparente ayant une surface non spéculaire est présente pour la visualisation de ladite surface optiquement active ;
et la mise en contact de ladite surface optiquement active avec un échantillon comprenant potentiellement ledit analyte auquel on s'intéresse dans des conditions dans lesquelles ledit analyte peut interagir avec ladite surface optiquement active de façon à faire présenter par ladite surface optiquement active ladite deuxième couleur d'interférence lorsque ledit analyte est présent.
